# EUROPÄISCHE PATENTSCHRIFT

(11) **EP 3 169 387 B1**
(45) Veröffentlichungstag und Bekanntmachung des Hinweises auf die Patenterteilung: **29.08.2018**
(21) Anmeldenummer: 15756812.2
(22) Anmeldetag: 16.07.2015
(51) Int. Cl.: A61M 5/32, A61M 25/06

(54) **SICHERHEITSNADELANORDNUNG ZUR ENTNAHME VON FLÜSSIGKEIT AUS EINEM KÖRPER**
SAFETY NEEDLE ARRANGEMENT FOR DRAWING LIQUID FROM A BODY
ENSEMBLE D'AIGUILLE DE SÉCURITÉ POUR LE PRÉLÈVEMENT D'UN LIQUIDE D'UN CORPS

(30) Priorität: 18.07.2014 AT 505002014
(43) Veröffentlichungstag der Anmeldung: 24.05.2017
(73) Patentinhaber: Greiner Bio-One GmbH, 4550 Kremsmünster (AT)
(72) Erfinder: BAUER, Christian, A-4550 Kremsmünster (AT); EBETSBERGER, Franz, A-4550 Kremsmünster (AT); KIRCHMEIR, Franz, A-4030 Linz (AT); MAYR, Andreas, A-4522 Sierning (AT); SCHIMPL, Melanie Christina, A-4020 Linz (AT); STRASSER, Gerhard, A-4550 Kremsmünster (AT)
(74) Vertreter: Burger, Hannes
(86) Internationale Anmeldenummer: PCT/AT2015/050169
(87) Internationale Veröffentlichungsnummer: WO 2016/007981

(56) Entgegenhaltungen:
- WO-A2-02/26284
- US-A1- 2007 016 148

## Beschreibung

Die Erfindung betrifft eine Sicherheitsnadelanordnung zur Entnahme von Flüssigkeit aus einem Körper, wie dies im Anspruch 1 beschrieben ist.

Die US 2007/0016148 A1 beschreibt eine gattungsgemäße Sicherheitsnadelanordnung umfassend einen Basiskörper mit einem distalen Ende und einem proximalen Ende, wobei sich zwischen dem distalen Ende des Basiskörpers und dem proximalen Ende des Basiskörpers in diesem eine Durchgangsöffnung erstreckt. Weiters umfasst die Sicherheitsnadelanordnung einen Kanülenträger mit einem distalen Ende und einem proximalen Ende, wobei sich zwischen den beiden Enden des Kanülenträgers eine Durchströmöffnung erstreckt. Der Kanülenträger ist in der Durchgangsöffnung des Basiskörpers in Axialrichtung verstellbar aufgenommen. Eine Kanüle weist ein distales Ende und ein proximales Ende auf, wobei sich zwischen den beiden Enden der Kanüle innerhalb dieser ein Strömungskanal erstreckt, und das proximale Ende der Kanüle an dem distalen Ende des Kanülenträgers gehalten ist. Ein Stellelement ist zwischen dem Basiskörper und dem Kanülenträger wirkend angeordnet, wobei das Stellelement den Kanülenträger mitsamt der daran gehaltenen Kanüle von einer ersten Stellung, bei welcher die Kanüle über das distale Ende des Basiskörpers vorragt, in eine zweite Stellung selbsttätig verlagert, bei welcher zumindest das distale Ende der Kanüle vom Basiskörper abgedeckt ist. Eine Verriegelungsvorrichtung weist mehrere erste und zweite Verriegelungselemente auf. Durch die Verriegelungsvorrichtung ist bei einer sich in gegenseitigem Eingriff befindlichen Position der ersten und zweiten Verriegelungselemente die erste relative Stellung des Kanülenträgers mitsamt der Kanüle bezüglich des Basiskörpers festlegt. Die ersten Verriegelungselemente sind im Bereich des proximalen Endes des Basiskörpers und die zweiten Verriegelungselemente im Bereich des proximalen Endes des Kanülenträgers angeordnet. Nachteilig dabei ist, dass nach dem Entriegeln der Verriegelungsvorrichtung und dem damit verbundenen Halten des Kanülenträgers vom Stellelement eine Druckkraft in Axialrichtung auf den Basiskörper ausgeübt wird und dadurch der Basiskörper in Richtung auf die Einstichstelle gedrückt wird. Dies führt zu einem zusätzlichen auf den Körper ausgeübten Schmerzreiz.

Aus der EP 0 830 871 B1 sowie der dazu parallelen US 5,928,199 A ist eine ähnlich aufgebaute Sicherheitsnadelanordnung bekannt geworden, wie diese zuvor detailliert zur US 2007/0016148 A1 beschrieben worden ist. Diese Sicherheitsnadelanordnung weist hingegen kein selbsttätig wirkendes Stellelement für die Rückstellung der Kanüle in eine diese abdeckende Stellung auf. Nach dem Entriegeln der zwischen dem Basiskörper und dem Kanülenträger angeordneten Verriegelungsvorrichtung ist der Kanülenträger mitsamt der Kanüle von der Bedienperson durch händisches Zurückziehen in die die Kanüle abdeckende zweite Stellung zu verbringen.

Der vorliegenden Erfindung liegt die Aufgabe zugrunde, eine nach der Entriegelung einer Verriegelungsvorrichtung selbsttätig in eine Schutzstellung rückstellbare Sicherheitsnadelanordnung zu schaffen, bei welcher die Einstichstelle bzw. der Einstichbereich in den Körper im Zuge der Entfernung und der nachfolgenden Wegnahme der Sicherheitsnadelanordnung vom Körper kein unnötiger zusätzlicher Schmerzreiz verursacht wird.

Diese Aufgabe der Erfindung wird durch die Merkmale des Anspruches 1 gelöst. Die durch die Merkmale des Anspruches 1 erzielten Vorteile liegen darin, dass die durch die Vorsprünge gebildeten, ersten Verriegelungselemente im Bereich des proximalen Endes des Basiskörpers angeordnet und an diesem für die Entriegelung der Verriegelungsvorrichtung auch zu betätigen sind. Damit kann mit einer einfachen Einhandbedienung nach dem Einsatz der Sicherheitsnadelanordnung diese entriegelt und nachfolgend die gebrauchte Kanüle in die Schutzstellung verlagert werden. So ist die Entriegelung der sich in Eingriff befindlichen Verriegelungselemente bewusst durch eine Bedienperson auszulösen, wobei die weitere Verstellung in die Schutzstellung selbsttätig durch das vorgespannt angeordnete Stellelement erfolgt. Ein weiterer Vorteil besteht noch darin, dass durch die einander diametral gegenüber liegend angeordneten ersten Verriegelungselemente am Basiskörper diese beispielsweise durch den Daumen und den Zeigefinger der Bedienperson gleichzeitig zu betätigen sind, wodurch ein seitliches Wegdrücken und damit verbunden eine relative Verlagerung der Kanüle innerhalb des Körpers vermieden wird. Durch diese zentrale Auslösung und der nachfolgenden selbsttätigen Rückstellung des Kanülenträgers mitsamt der Kanüle in die Schutzstellung ist kein zusätzlicher Bedienaufwand mehr notwendig. Dadurch, dass der Basiskörper ortsfest relativ gegenüber dem Körper verbleibt, kommt es zu keiner zusätzlichen Druckbelastung nach dem Auslösen des Stellelements auf die Einstichstelle. Es wird lediglich die Kanüle in die Schutzstellung, nämlich in die abdeckende Stellung innerhalb des Basiskörpers, verlagert und damit ohne jegliches Zutun der Bedienperson eine sichere Position erreicht, wodurch Stichverletzungen im Bereich der gebrauchten Kanüle verhindert sind.

Vorteilhaft ist auch eine weitere Ausführungsform nach Anspruch 2, da dadurch eine ausreichende Federwirkung durch die Haltearme auf die dort jeweils angeordneten Vorsprünge ausgeübt werden kann, um so die Einsatzstellung der Kanüle in einer sicheren Verrastungsstellung der zusammenwirkenden Verriegelungselemente zu bewirken. Damit können unbedachte Auslösungen der Verriegelungsvorrichtung verhindert werden.

Vorteilhaft ist weiters eine Ausbildung nach Anspruch 3, da dadurch eine beidseits in Richtung auf die Längsachse gerichtete Auslösebewegung erzielt werden kann. Dadurch kann durch ein einfaches zentrisches Zusammendrücken der beiden Haltearme die Entriegelung der miteinander in Eingriff stehenden ersten und zweiten Verriegelungselemente erreicht werden.

Durch die Ausbildung nach Anspruch 4 ist es möglich, die Verriegelung bzw. Verrastung der miteinander in Eingriff stehenden ersten und zweiten Verriegelungselemente solange sicherstellen zu können, bis dass von der Bedienperson die entsprechende Entriegelung erfolgt. Durch das jeweilige Hineinragen der Vorsprünge in die dafür vorgesehenen Ausnehmungen kann so aber auch eine ungewollte Auslösung der Verriegelungsvorrichtung verhindert werden.

Nach einer anderen Ausführungsvariante gemäß Anspruch 5 wird so die Möglichkeit geschaffen, die in Axialrichtung ausgerichtet verlaufenden Haltearme mitsamt den daran angeordneten Vorsprüngen innerhalb des Halteansatzes anordnen zu können und so einen geschützten Verriegelungsbereich für die miteinander in Eingriff stehenden ersten und zweiten Verriegelungselemente zu schaffen.

Vorteilhaft ist auch eine Weiterbildung nach Anspruch 6, da dadurch für das Stellelement in seiner vorgespannten Stellung ein Aufnahmeraum geschaffen werden kann. Dadurch kann das Stellelement in der Einsatzstellung vor einer Manipulation von außen sicher geschützt abgedeckt werden. Damit kann nach der Auslösung der Verriegelungsvorrichtung stets eine automatisierte, selbsttätige Rückstellung des Kanülenträgers mitsamt der daran angeordneten Kanüle in die Schutzstellung innerhalb des Basiskörpers erreicht werden.

Bei der Ausgestaltung nach Anspruch 7 ist von Vorteil, dass so nach dem Gebrauch der Sicherheitsnadelanordnung eine erneute Wiederverwendung gesichert verhindert werden kann. Damit können nicht nur ungewollte Stichverletzungen durch die bereits gebrauchte Kanüle verhindert werden, sondern bei einer Wiederverwendung auch eine mögliche Übertragung bzw. Ansteckung ausgehend von der gebrauchten Kanüle vermieden werden.

Durch die Weiterbildung nach Anspruch 8 wird erreicht, dass so eine bessere Lagefixierung der gesamten Sicherheitsnadelanordnung während des bestimmungsgemäßen Gebrauchs am Körper erreicht werden kann.

Es ist aber auch eine Ausbildung, wie im Anspruch 9 beschrieben, möglich, da dadurch nicht nur eine ausreichende Fixiermöglichkeit am Körper geschaffen werden kann, sondern auch eine dazu zentrische und in etwa parallel dazu ausgerichtete Auslösebewegung für die Verriegelungsvorrichtung festgelegt wird.

Durch die Ausbildung gemäß einem der Ansprüche 10 oder 11 kann eine Reduzierung der Verstellgeschwindigkeit, nämlich der Relativgeschwindigkeit zwischen dem nahezu bis vollständig ortsfesten Basiskörper und dem Kanülenträger erzielt werden. Das oder die Bremselemente dienen dazu, die rasche Beschleunigung zu dämpfen, sodass es dadurch zumindest am Beginn des Verstellvorgangs zum Aufbau einer geringfügigen Rückhaltekraft kommt und die Verstellkraft des Stellelements diese Rückhaltekraft überwinden muss.

Es ist auch eine Ausgestaltung nach Anspruch 12 von Vorteil. Dadurch wird der hohen Beschleunigung am Beginn des Verstellvorgangs bzw. des Verstellwegs entgegengewirkt.

Zum besseren Verständnis der Erfindung wird diese anhand der nachfolgenden Figuren näher erläutert.

Es zeigen jeweils in stark vereinfachter, schematischer Darstellung:
- Fig. 1: eine Sicherheitsnadelanordnung in ihrer Einsatzstellung, in schaubildlicher Darstellung;
- Fig. 2: die Sicherheitsnadelanordnung nach Fig. 1, jedoch in ihrer Schutzstellung der Kanüle, in schaubildlicher Darstellung, teilweise geschnitten;
- Fig. 3: die Sicherheitsnadelanordnung nach den Fig. 1 und 2 in einer Art Explosionsdarstellung mit voneinander distanzierten Einzelkomponenten;
- Fig. 4: den Kanülenträger mit Kanüle sowie Halteansatz, im Axialschnitt.

Einführend sei festgehalten, dass in den unterschiedlich beschriebenen Ausführungsformen gleiche Teile mit gleichen Bezugszeichen bzw. gleichen Bauteilbezeichnungen versehen werden, wobei die in der gesamten Beschreibung enthaltenen Offenbarungen sinngemäß auf gleiche Teile mit gleichen Bezugszeichen bzw. gleichen Bauteilbezeichnungen übertragen werden können. Auch sind die in der Beschreibung gewählten Lageangaben, wie z.B. oben, unten, seitlich usw. auf die unmittelbar beschriebene sowie dargestellte Figur bezogen und sind diese Lageangaben bei einer Lageänderung sinngemäß auf die neue Lage zu übertragen.

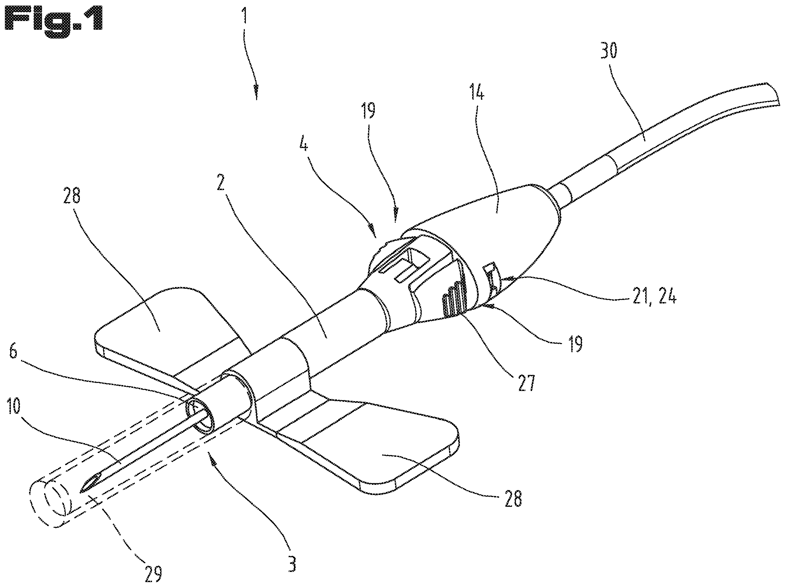

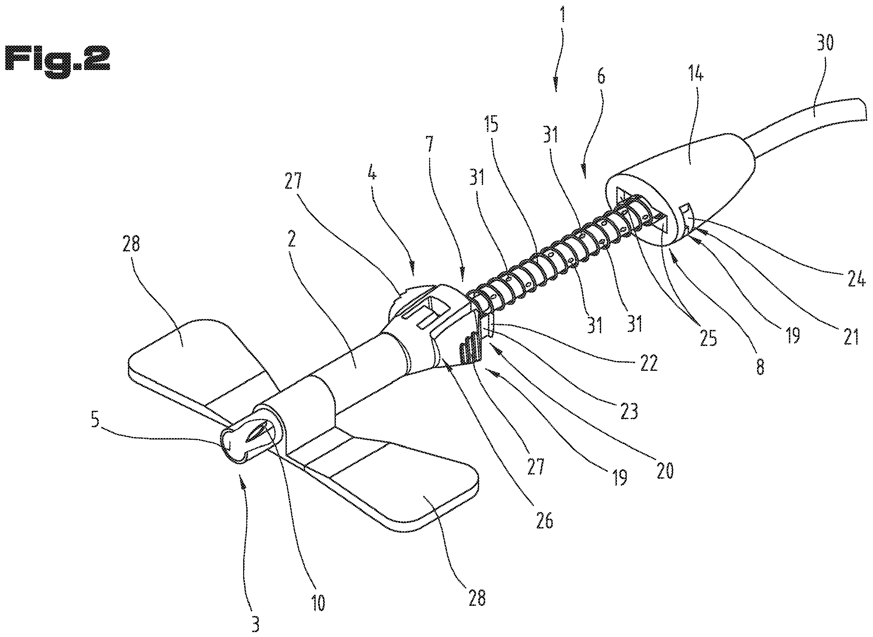

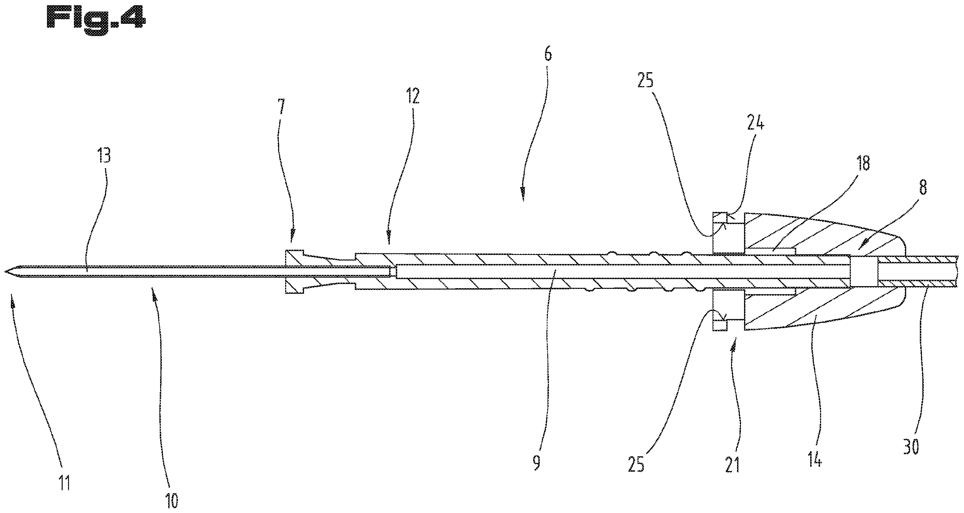

In den Fig. 1 bis 4 ist eine Sicherheitsnadelanordnung 1 gezeigt, welche in der Medizintechnik Verwendung findet. Die Sicherheitsnadelanordnung 1 kann beispielsweise zur Entnahme von Flüssigkeit, insbesondere Blut, aus einem Körper oder zum Zuführen von Flüssigkeit in den Körper eingesetzt werden. Eine derartig ausgebildete Sicherheitsnadelanordnung 1 kann auch als sogenannter "Butterfly" bezeichnet werden. Die Fig. 1 und 2 zeigen die Sicherheitsnadelanordnung 1 in deren zusammengebauten Zustand, jedoch in zueinander unterschiedlichen Stellungen von einzelnen Bauteilkomponenten zueinander.

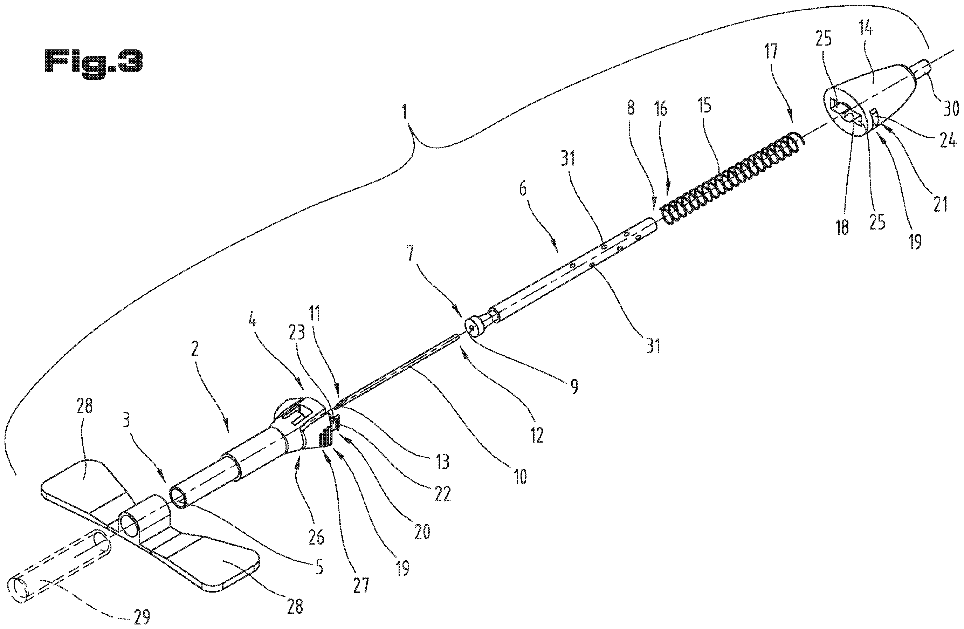

In der Fig. 3 sind die einzelnen Bauteile bzw. Bauteilkomponenten der Sicherheitsnadelanordnung 1 in einer voneinander getrennten Anordnung gezeigt, um deren Ausbildung besser ersehen zu können. So kann die Sicherheitsnadelanordnung 1 einen Basiskörper 2 mit einem distalen Ende 3 und einem proximalen Ende 4 umfassen. Bevorzugt ist der Basiskörper 2 rohrförmig, insbesondere zylinderförmig, ausgebildet und weist in Richtung seiner Axialerstreckung eine sich zwischen dem distalen Ende 3 des Basiskörpers 2 und dem proximalen Ende 4 des Basiskörper 2 erstreckende Durchgangsöffnung 5 auf.

Weiters kann die Sicherheitsnadelanordnung 1 auch noch einen Kanülenträger 6 umfassen, welcher seinerseits ein distales Ende 7 sowie ein in Axialrichtung davon distanziertes proximales Ende 8 aufweist. Im Kanülenträger 6 erstreckt sich zwischen dem distalen Ende 7 und dem proximalen Ende 8 eine Durchströmöffnung 9. Des Weiteren ist der Kanülenträger 6 derart ausgebildet, dass dieser in der Durchgangsöffnung 5 des Basiskörpers 2 in Axialrichtung verstellbar aufgenommen, insbesondere geführt ist.

Des Weiteren kann die Sicherheitsnadelanordnung 1 auch noch eine Kanüle 10 umfassen, welche ihrerseits ein distales Ende 11 sowie ein in Axialrichtung davon distanziertes proximales Ende 12 aufweist. Innerhalb der Kanüle 10 erstreckt sich zwischen dem distalen Ende 11 und dem proximalen Ende 12 ein Strömungskanal 13. Weiters kann das proximale Ende 12 der Kanüle 10 an dem distalen Ende 7 des Kanülenträgers 6 gehalten, insbesondere daran feststehend befestigt sein. Die Verbindung des proximalen Endes 12 der Kanüle 10 mit dem Kanülenträger 6 kann z.B. durch eine zumindest luftdichte Klebeverbindung erfolgen. Es wäre unabhängig davon aber auch noch möglich, anstatt der Klebeverbindung oder zusätzlich zu dieser, einen Presssitz auszubilden. Dazu ist ein entsprechender Abmessungsunterschied zwischen dem Endbereich der Kanüle 10 und der Aufnahmeöffnung im Kanülenträger 6 auszubilden.

Damit kann ausgehend vom distalen Ende 11 der Kanüle 10 ein Durchfluss an Flüssigkeit durch den Strömungskanal 13 und weiters durch die Durchströmöffnung 9 im Kanülenträger 6 oder auch in dazu entgegengesetzter Richtung erzielt werden.

Weiters kann der Kanülenträger 6 im Bereich seines proximalen Endes 8 auch noch einen zusätzlichen Halteansatz 14 aufweisen. Der Halteansatz 14 kann entweder durch einen eigenen Bauteil gebildet sein oder aber auch integraler Bestandteil des Kanülenträgers 6 sein. Handelt es sich beispielsweise, wie im vorliegenden Ausführungsbeispiel, um jeweils eigene Bauteile, kann sich das proximale Ende 8 des Kanülenträgers 6 in Axialrichtung gesehen in den Halteansatz 14 hinein erstrecken und ist dort mit diesem verbunden. Auch hier kann die gegenseitige Halterung bzw. Verbindung zwischen dem proximalen Ende 8 des Kanülenträgers 6 und dem Halteansatz 14 kann z.B. durch eine zumindest luftdichte Klebeverbindung erfolgen. Es wäre unabhängig davon aber auch noch möglich, anstatt der Klebeverbindung oder zusätzlich zu dieser, einen Presssitz auszubilden. Dazu ist ein entsprechender Abmessungsunterschied zwischen dem Endbereich des Kanülenträgers 6 und dem Halteansatz 14 auszubilden.

Zur selbsttätigen Verlagerung des Kanülenträgers 6 relativ bezüglich des Basiskörpers 2 kann die Sicherheitsnadelanordnung 1 auch noch ein eigenes Stellelement 15 umfassen, welches im Einbauzustand zwischen dem Basiskörper 2 und dem Kanülenträger 6 wirkend angeordnet ist. Im vorliegenden Ausführungsbeispiel ist das Stellelement 15 durch eine Spiraldruckfeder gebildet, welche sich an der Außenseite des Kanülenträgers 6 erstreckend angeordnet ist. Ein dem Basiskörper 2 zugewendetes distales Stellelementende 16 ist im Bereich des proximalen Endes 4 des Basiskörpers 2 an diesem abgestützt. Das Stellelement 15 kann sich auch noch in Axialrichtung über eine Teillänge in den Basiskörper 2 hinein erstrecken.

Im vorliegenden Ausführungsbeispiel ist das weitere in Axialrichtung davon distanzierte proximale Stellelementende 17 am Halteansatz 14 abgestützt. Um in der zusammengeschobenen Einsatzposition bzw. vorgespannten Stellung des Stellelementes 15 ausreichend Platz dafür zu haben, kann es vorteilhaft sein, wenn sich das Stellelement 15 über eine Teillänge auf der dem Basiskörper 2 zugewendeten Seite in den Halteansatz 14 hinein erstrecken kann. Dazu ist im Halteansatz 14 auf seiner dem Basiskörper 2 zugewendeten Seite eine sich über eine Teillänge zwischen dem Kanülenträger 6 und dem Halteansatz 14 erstreckende Aufnahmeöffnung 18 zur Aufnahme des Stellelements 15 angeordnet. Die Querschnittsform der Aufnahmeöffnung 18 ist dabei an die Abmessungen des Stellelements 15 anzupassen. Im vorliegenden Ausführungsbeispiel ist die Aufnahmeöffnung 18 beispielsweise rohrförmig bzw. hohlzylinderförmig ausgebildet.

In der Fig. 1 ist dabei die erste relative Stellung des Kanülenträgers 6 mitsamt der daran gehaltenen Kanüle 10 gezeigt. In dieser ersten Stellung ragt die Kanüle 10 über das distale Ende 3 des Basiskörpers 2 vor.

In der Fig. 2 ist die sogenannte Schutzstellung gezeigt, welche hier als zweite Stellung bezeichnet wird. In dieser zweiten Stellung ist zumindest das distale Ende 11 der Kanüle 10 vom Basiskörper 2 abgedeckt, um so Stichverletzungen durch die gebrauchte Kanüle zu vermeiden. Die Verlagerung, insbesondere die Axialverstellung, des Kanülenträgers 6 mitsamt der daran gehaltenen Kanüle 10 erfolgt durch das zuvor beschriebene Stellelement 15 selbsttätig.

Um den Kanülenträger 6 mitsamt der daran gehaltenen Kanüle 10 in der ersten Stellung relativ bezüglich des Basiskörpers 2 verrastet bzw. verriegelt halten zu können, ist eine eigene Verriegelungsvorrichtung 19 vorgesehen, welche ebenfalls Bestandteil der Sicherheitsnadelanordnung 1 sein kann. Die Verriegelungsvorrichtung 19 umfasst bei diesem Ausführungsbeispiel mehrere erste Verriegelungselemente 20 sowie mehrere zweite Verriegelungselemente 21. In der Verriegelungsstellung der Verriegelungsvorrichtung 19 wird durch die in Eingriff befindlichen ersten und zweiten Verriegelungselemente 20, 21 die erste relative Stellung des Kanülenträgers 6 mitsamt der Kanüle 10 bezüglich des Basiskörpers 2 festgelegt.

Die ersten Verriegelungselemente 20 sind im vorliegenden Ausführungsbeispiel durch einander diametral gegenüberliegend angeordnete Vorsprünge 22 gebildet und sind im Bereich des proximalen Endes 4 des Basiskörpers 2 an diesem angeordnet oder ausgebildet. Weiters können die Vorsprünge 22 jeweils an einem in Radialrichtung verstellbaren Haltearm 23 angeordnet sein.

Die zweiten Verriegelungselemente 21 sind im vorliegenden Ausführungsbeispiel durch Ausnehmungen 24 gebildet, welche ebenfalls einander diametral gegenüberliegend angeordnet sind, um einen Eingriff mit den zuvor beschriebenen ersten Verriegelungselementen 20 der Verriegelungsvorrichtung 19 zu ermöglichen. Die zweiten Verriegelungselemente 21 sind dabei im Bereich des proximalen Endes 8 des Kanülenträgers 6 angeordnet. Im vorliegenden Ausführungsbeispiel sind im Halteansatz 14 die die zweiten Verriegelungselemente 21 bildenden Ausnehmungen 24 angeordnet oder ausgebildet.

Zur Aufnahme der Haltearme 23 bei sich in der ersten Stellung befindlichem Kanülenträger 6 kann der Halteansatz 14 im Bereich seines dem Basiskörper 2 zugewendeten distalen Endes diametral zueinander angeordnete Einsteckschlitze 25 aufweisen. Damit können die Haltearme 23 bei sich in der ersten Stellung befindlichem Kanülenträger 6 in diese Einsteckschlitze 25 hineinragen und die Vorsprünge 22 in rastendem bzw. verriegelnden Eingriff mit den Ausnehmungen 24 stehen.

Um die ersten Verriegelungselemente 20 - im vorliegenden Ausführungsbeispiel die Vorsprünge 22 - in radialer Richtung bezüglich der Längserstreckung des Basiskörpers 2 relativ zu diesem verlagern zu können, können die Haltearme 23 jeweils auf der von den daran angeordneten Vorsprüngen 22 abgewendeten Seite an Anlenkungsabschnitten 26 mit dem Basiskörper 2 gelenkig, insbesondere federnd, verbunden sein. Die Vorsprünge 22 können derart an den Haltearmen 23 angeordnet oder ausgebildet sein, dass diese jeweils auf die von der im Basiskörper 2 angeordneten Durchgangsöffnung 5 abgewendeten Seite von den Haltearmen 23 in radialer Richtung darüber vorragen. Des Weiteren können die Haltearme 23 in ihrer Axialerstreckung zwischen deren Anlenkungsabschnitten 26 und den Vorsprüngen 22 jeweils distanziert vom Basiskörper 2 angeordnet sein. Diese Distanzierung der Haltearme 23 vom Basiskörper 2 kann beispielsweise durch die Ausbildung oder Anordnung von Schlitzen erfolgen, welche jeweils im Bereich der Anlenkungsabschnitte 26 enden.

Um eine bessere Betätigung der Haltearme 23 durchführen zu können, kann an den Haltearmen 23 jeweils zwischen deren Anlenkungsabschnitten 26 und den Vorsprüngen 22 ein zusätzlicher Betätigungsansatz 27 angeordnet oder ausgebildet sein. Dieser erleichtert die Betätigung der Haltearme 23 in radialer Richtung auf die Längsachse. Sind, wie im vorliegenden Ausführungsbeispiel die Haltearme 23 mit den gegebenenfalls daran angeordneten oder ausgebildeten Betätigungsansätzen 27 diametral einander gegenüberliegend angeordnet, kann eine unbeabsichtigte Auslösung der Verriegelungsvorrichtung 19 leichter verhindert werden. Zur Auslösung ist eine bewusst durchzuführende Verstellung beider Haltearme 23 in die Auslösestellung notwendig. Bevorzugt erfolgt die Auslösung nahezu gleichzeitig.

Zur Vermeidung von Fehlauslösungen der Verriegelungsvorrichtung 19 kann eine Außenabmessung des Halteansatzes 14 im Bereich seiner dem Basiskörper 2 zugewendeten Seite in etwa der Außenabmessung der Betätigungsansätze 27 entsprechen. Damit kann ein Überragen der Betätigungsansätze 27 über den Halteansatz 14 vermieden werden.

Durch diese radiale Verstellung bzw. Verlagerung der Vorsprünge 22 kommen diese außer Eingriff mit den Ausnehmungen 24, wodurch die Verriegelungsvorrichtung 19 außer Eingriff kommt. Sobald der Eingriff der Verriegelungsvorrichtung 19 gelöst ist, kommt das Stellelement 15 zur Wirkung und verlagert selbsttätig und automatisch den Kanülenträger 6 und somit auch die Kanüle 10 in deren abgedeckte Stellung.

Um eine neuerliche Verwendung der Sicherheitsnadelanordnung 1 zu vermeiden, kann der Kanülenträger 6 in seiner zweiten relativen Stellung bezüglich des Basiskörpers 2 mittels einer im Bereich des proximalen Endes 4 des Basiskörpers 2 angeordneten Arretiervorrichtung in Axialrichtung relativ bezüglich des Basiskörpers arretiert an diesem gehaltert sein. Diese Arretiervorrichtung kann derart ausgebildet sein, dass in der zweiten Stellung des Kanülenträgers 6 dieser in Axialrichtung gesehen, in beide Richtungen an einer erneuten Verstellung gehindert ist. Damit kann verhindert werden, dass der Kanülenträger 6 sich vom Basiskörper 2 löst und gleichzeitig auch eine erneute Rückstellung des Kanülenträgers 6 in die erste Stellung verhindert ist.

Weiters können am Basiskörper 2 von diesem in Radialrichtung abstehende Flügel 28 angeordnet oder ausgebildet sein. Die Flügel 28 können in einer aneinander anliegenden Stellung während des Einstichvorgangs zur besseren Halterung durch die Bedienperson dienen. Ist die Sicherheitsnadelanordnung 1 mit ihrer Kanüle 10 für die Entnahme von Flüssigkeit aus dem Körper oder zum Zuführen von Flüssigkeiten in den Körper eingestochen, kann die gesamte Sicherheitsnadelanordnung 1 mit den Flügeln 28 mit einem zusätzlichen Klebestreifen oder einem anderen Haltemittel an der nicht näher dargestellten Oberfläche des Körpers gehalten bzw. fixiert werden.

Weiters können die Flügel 28 jeweils in gleiche Richtung vom Basiskörper 2 vorragen, als die diametral zueinander an den Haltearmen 23 angeordneten Vorsprünge 22. Damit kann eine eindeutige Ausrichtung der ersten Verriegelungselemente 20 relativ bezüglich der Oberfläche, nämlich der Haut des Körpers, erzielt werden.

Da am Beginn der Verstellbewegung das zumeist stark vorgespannte Stellelement 15 mit voller Kraft und damit bedingt mit hoher Beschleunigung die Verstellbewegung einleitet, kann die rasche automatische Verstellbewegung dazu führen, dass Körperflüssigkeit, insbesondere Blut, in Tröpfchenform weggeschleudert wird. Damit kann ein erhöhtes Ansteckungsrisiko entstehen.

Zur Reduzierung der Verstellgeschwindigkeit, nämlich der Relativgeschwindigkeit zwischen dem nahezu bis vollständig ortsfesten Basiskörper 2 und dem Kanülenträger 6 ist in den Fig. 2 bis 4 noch vereinfacht angedeutet, dass an der Oberfläche des bevorzugt rohrförmigen Kanülenträgers 6 zumindest ein als Vorsprung oder Ansatz ausgebildetes Bremselement 31 angeordnet oder ausgebildet ist. Das oder die Bremselemente 31 dienen dazu, die rasche Beschleunigung zu dämpfen. Diese Brems- bzw. Dämpfungswirkung kann auch durch Erhöhung der Reibung zwischen dem Basiskörper 2 und dem Kanülenträger 6 erreicht werden. Je nach Ausbildung, Anordnung und/oder Anzahl der Bremselemente 31 kann die Brems- bzw. Dämpfungswirkung festgelegt werden.

Im vorliegenden Ausführungsbeispiel sind lediglich einige Bremselemente 31 im Bereich des proximalen Endes 8 des Kanülenträgers 6 angeordnet oder ausgebildet, wie dies vereinfacht stilisiert dargestellt worden ist. Dadurch wird der hohen Beschleunigung am Beginn des Verstellvorgangs bzw. des Verstellwegs entgegengewirkt. Die bevorzugt über den Kanülenträger 6 vorragenden Bremselemente 31 wirken mit der Durchgangsöffnung 5 im Basiskörper 2 zusammen, sodass es dadurch zumindest am Beginn des Verstellvorgangs zum Aufbau einer geringfügigen Rückhaltekraft kommt und die Verstellkraft des Stellelements 15 diese Rückhaltekraft überwinden muss.

Es wäre aber auch noch möglich, wie dies in der Fig. 2 noch in strichlierten Linien dargestellt ist, das oder die Bremselemente 31 nicht nur für den Beginn des Verstellvorgangs des Kanülenträgers 6 an diesem anzuordnen oder auszubilden, sondern über die gesamte axiale Baulänge vorzusehen. Dabei kann auch die Anzahl und/oder die Bauhöhe und damit das Überragen des Kanülenträgers 6 beeinflusst werden. So kann bei geringerer Anzahl und/oder geringerem Überstand der Bremselemente 31 als bei jenen Bremselemente 31, welche am Beginn des Verstellvorgangs zur Wirkung kommen, die Bremswirkung während des Verstellwegs reduziert werden.

Die Entnahme von Flüssigkeit, insbesondere Blut, aus dem Körper oder das Zuführen von Flüssigkeit in den Körper kann mittels der zuvor beschriebenen Sicherheitsnadelanordnung 1 mit den nachfolgenden Schritten durchgeführt werden. Dazu kann am proximalen Ende 8 des Kanülenträgers 6 und/oder des Halteansatzes 14 ein Schlauch 30 mit seinem ersten Ende angeordnet bzw. befestigt sein. Am nicht dargestellten anderen Ende können die unterschiedlichsten Anschlüsse oder Vorrichtungen befestigt oder damit gekuppelt sein.

Dabei sei erwähnt, dass die Reihenfolge der angeführten Schritte nicht zwingend einzuhalten ist, sondern die einzelnen Schritte auch in zueinander unterschiedlicher Reihenfolge durchgeführt werden können.

Die Sicherheitsnadelanordnung 1 ist im umgebauten Zustand zumeist in einer sterilen Verpackung abgepackt und wird aus dieser durch eine Bedienperson aus dieser entnommen. Dabei befindet sich der Kanülenträger 6 mitsamt der daran gehaltenen Kanüle 10 in der ersten Stellung, bei welcher die Kanüle 10 das distale Ende 3 des Basiskörpers 2 überragt. Um in diesem Zustand eine Stichverletzung zu vermeiden, ist in der Fig. 1 in strichlierten Linien eine zusätzliche, bevorzugt rohrförmig ausgebildete, Schutzhülle 29 angedeutet. Die Schutzhülle 29 kann nur durch einen rohrförmigen, an beiden Enden offenen Bauteil gebildet sein, wobei dann die gesamte Sicherheitsnadelanordnung 1 in einer zusätzlichen Überverpackung, z.B. einer nicht dargestellten Blisterhülle, bis zur bestimmungsgemäßen Verwendung steril verpackt ist.

Die Schutzhülle 29 kann auch noch im Bereich ihres vom Basiskörper 2 abgewendeten distalen Endes dicht verschlossen ausgebildet sein, wie dies hier bei der in strichlierten Linien dargestellten Schutzhülle 29 gezeigt ist. Dies kann z.B. durch eine Abschlusswand erfolgen. Bei einer derartigen Ausbildung der Schutzhülle 29 kann diese auch als Schutzkappe bezeichnet werden. Damit wird die Möglichkeit geschaffen, zumindest die Kanüle 10 vor deren bestimmungsgemäßen Erstgebrauch steril aufbewahren zu können und dabei auf eine ansonsten übliche Überverpackung, beispielsweise in Form einer bakteriendichten Blisterhülle, überhaupt verzichten zu können. So kann Verpackungsmaterial eingespart werden und trotzdem die Sterilität der Kanüle 10 bis zu deren Gebrauch sichergestellt werden.

Nach Entfernung dieser Schutzhülle 29 oder der Schutzkappe vom Basiskörper 2 liegt die Kanüle 10 frei und kann an der dafür vorgesehenen Einstichstelle in den Körper eingestochen werden.

In weiterer Folge kann die ordnungsgemäße Lage der Kanüle 10 im Körper festgestellt werden. In einem weiteren Schritt kann das Abnehmen der Flüssigkeit, insbesondere Blut, aus dem Körper erfolgen. Unabhängig davon wäre aber auch das Zuführen von Flüssigkeit in den Körper durch die Sicherheitsnadelanordnung 1 hindurch möglich.

Das Entfernen der Kanüle 10 aus der Einstichstelle des Körpers erfolgt dadurch, dass der Basiskörper 2 der Sicherheitsnadelanordnung 1 ortsfest relativ bezüglich des Körpers gehalten wird. Anschließend wird die in Eingriff befindliche Verriegelungsvorrichtung 19 zwischen dem Basiskörper 2 und dem Kanülenträger 6 außer Eingriff gebracht. Dies erfolgt dadurch, dass gleichzeitig die einander diametral gegenüberliegend am Basiskörper angeordneten Vorsprünge 22 der Verriegelungsvorrichtung 19 in Richtung auf die Längsachse des Basiskörpers 2 verstellt werden. Dabei kommen die Vorsprünge 22 außer Eingriff mit dem im Kanülenträger 6, insbesondere dessen Halteansatz 14, angeordneten Ausnehmungen 24. Nach der Entriegelung der Verriegelungsvorrichtung 19 wird der Kanülenträger 6 mitsamt der daran gehaltenen Kanüle 10 mittels des Stellelements 15 selbsttätig in die zweite Stellung verlagert. Dabei kann die Kanüle 10 automatisch aus der Einstichstelle herausgezogen werden und wird in weiterer Folge in die zuvor beschriebene zweite relative Stellung verlagert. Dabei wird in dieser zweiten Stellung zumindest das distale Ende 11 der Kanüle 10 vom Basiskörper 2 abgedeckt. Während dieses selbststätig durchgeführten Rückstellvorgangs, welcher nur durch die Bedienperson auszulösen ist, kann der Basiskörper 2 in seiner Lage unverändert am Körper verbleiben. Dadurch werden keine unnötigen relativen Verlagerungen der Sicherheitsnadelanordnung 1 bei sich noch in der Einstichstelle befindlicher Kanüle 10 durchgeführt.

Ist die Schutzstellung erreicht, kann die Abnahme bzw. das Wegnehmen der gesamten Sicherheitsnadelanordnung 1 vom Körper durchgeführt werden. Anschließend daran wird die benutzte Sicherheitsnadelanordnung 1 entsprechend den Sicherheitsvorschriften entsorgt, wobei auch noch auf eine entsprechende sichere Aufbewahrung bis zur Entsorgung zu achten ist.

Das Ausführungsbeispiel zeigt eine mögliche Ausführungsvariante der Sicherheitsnadelanordnung 1, wobei an dieser Stelle bemerkt sei, dass die Erfindung nicht auf die speziell dargestellten Ausführungsvarianten derselben eingeschränkt ist, sondern vielmehr auch diverse Kombinationen der einzelnen Ausführungsvarianten untereinander möglich sind und diese Variationsmöglichkeit aufgrund der Lehre zum technischen Handeln durch gegenständliche Erfindung im Können des auf diesem technischen Gebiet tätigen Fachmannes liegt.

Weiters können auch Einzelmerkmale oder Merkmalskombinationen aus den gezeigten und beschriebenen unterschiedlichen Ausführungsbeispielen für sich eigenständige, erfinderische oder erfindungsgemäße Lösungen darstellen.

Die den eigenständigen erfinderischen Lösungen zugrundeliegende Aufgabe kann der Beschreibung entnommen werden.

Der Ordnung halber sei abschließend darauf hingewiesen, dass zum besseren Verständnis des Aufbaus der Sicherheitsnadelanordnung 1 diese bzw. deren Bestandteile teilweise unmaßstäblich und/oder vergrößert und/oder verkleinert dargestellt wurden.

### Bezugszeichenaufstellung

| | | | |
|---|---|---|---|
| 1 | Sicherheitsnadelanordnung | 31 | Bremselement |
| 2 | Basiskörper | | |
| 3 | distales Ende | | |
| 4 | proximales Ende | | |
| 5 | Durchgangsöffnung | | |
| 6 | Kanülenträger | | |
| 7 | distales Ende | | |
| 8 | proximales Ende | | |
| 9 | Durchströmöffnung | | |
| 10 | Kanüle | | |
| 11 | distales Ende | | |
| 12 | proximales Ende | | |
| 13 | Strömungskanal | | |
| 14 | Halteansatz | | |
| 15 | Stellelement | | |
| 16 | distales Stellelementende | | |
| 17 | proximales Stellelementende | | |
| 18 | Aufnahmeöffnung | | |
| 19 | Verriegelungsvorrichtung | | |
| 20 | erstes Verriegelungselement | | |
| 21 | zweites Verriegelungselement | | |
| 22 | Vorsprung | | |
| 23 | Haltearm | | |
| 24 | Ausnehmung | | |
| 25 | Einsteckschlitz | | |
| 26 | Anlenkungsabschnitt | | |
| 27 | Betätigungsansatz | | |
| 28 | Flügel | | |
| 29 | Schutzhülle | | |
| 30 | Schlauch | | |

## Patentansprüche

1. Sicherheitsnadelanordnung (1) umfassend einen Basiskörper (2) mit einem distalen Ende (3) und einem proximalen Ende (4), wobei sich zwischen dem distalen Ende (3) des Basiskörpers (2) und dem proximalen Ende (4) des Basiskörpers (2) in diesem eine Durchgangsöffnung (5) erstreckt, einen Kanülenträger (6) mit einem distalen Ende (7) und einem proximalen Ende (8), wobei sich zwischen dem distalen Ende (7) des Kanülenträgers (6) und dem proximalen Ende (8) des Kanülenträgers (6) eine Durchströmöffnung (9) erstreckt, und der Kanülenträger (6) in der Durchgangsöffnung (5) des Basiskörpers (2) in Axialrichtung verstellbar aufgenommen ist, eine Kanüle (10) mit einem distalen Ende (11) und einem proximalen Ende (12), wobei sich zwischen dem distalen Ende (11) der Kanüle (10) und dem proximalen Ende (12) der Kanüle (10) innerhalb der Kanüle (10) ein Strömungskanal (13) erstreckt, und das proximale Ende (12) der Kanüle (10) an dem distalen Ende (7) des Kanülenträgers (6) gehalten ist, ein Stellelement (15), welches zwischen dem Basiskörper (2) und dem Kanülenträger (6) wirkend angeordnet ist, und das Stellelement (15) den Kanülenträger (6) mitsamt der daran gehaltenen Kanüle (10) von einer ersten Stellung, bei welcher ersten Stellung die Kanüle (10) über das distale Ende (3) des Basiskörpers (2) vorragt, in eine zweite Stellung selbsttätig verlagert, bei welcher zweiten Stellung zumindest das distale Ende (11) der Kanüle (10) vom Basiskörper (2) abgedeckt ist, eine Verriegelungsvorrichtung (19) mit mehreren ersten und zweiten Verriegelungselementen (20, 21), welche Verriegelungsvorrichtung (19) bei einer sich in gegenseitigem Eingriff befindlichen Position der ersten und zweiten Verriegelungselemente (20, 21) die erste relative Stellung des Kanülenträgers (6) mitsamt der Kanüle (10) bezüglich des Basiskörpers (2) festlegt, und die ersten Verriegelungselemente (20) im Bereich des proximalen Endes (4) des Basiskörpers (2) und die zweiten Verriegelungselemente (21) im Bereich des proximalen Endes (8) des Kanülenträgers (6) angeordnet sind, **dadurch gekennzeichnet, dass** die ersten Verriegelungselemente (20) durch einander diametral gegenüberliegend angeordnete Vorsprünge (22) gebildet sind, welche Vorsprünge (22) jeweils an einem in Radialrichtung verstellbaren Haltearm (23) angeordnet sind, und die zweiten Verriegelungselemente (21) durch einander diametral gegenüberliegend angeordnete Ausnehmungen (24) gebildet sind.

2. Sicherheitsnadelanordnung (1) nach Anspruch 1, **dadurch gekennzeichnet, dass** die Haltearme (23) jeweils auf der von den daran angeordneten Vorsprüngen (22) abgewendeten Seite an Anlenkungsabschnitten (26) mit dem Basiskörper (2) gelenkig federnd verbunden sind.

3. Sicherheitsnadelanordnung (1) nach Anspruch 1 oder 2, **dadurch gekennzeichnet, dass** die Vorsprünge (22) jeweils auf die von der im Basiskörper (2) angeordneten Durchgangsöffnung (5) abgewendete Seite von den Haltearmen (23) in radialer Richtung vorragen und jeweils die Haltearme (23) in ihrer Axialerstreckung zwischen deren Anlenkungsabschnitten (26) und den Vorsprüngen (22) distanziert vom Basiskörper (2) angeordnet sind.

4. Sicherheitsnadelanordnung (1) nach einem der vorhergehenden Ansprüche, **dadurch gekennzeichnet, dass** am Kanülenträger (6) im Bereich seines proximalen Endes (8) ein Halteansatz (14) angeordnet oder ausgebildet ist, in welchem Halteansatz (14) die die zweiten Verriegelungselemente (21) bildenden Ausnehmungen (24) angeordnet sind.

5. Sicherheitsnadelanordnung (1) nach Anspruch 4, **dadurch gekennzeichnet, dass** der Halteansatz (14) im Bereich seines distalen Endes diametral gegenüberliegend angeordnete Einsteckschlitze (25) aufweist, in welche Einsteckschlitze (25) jeweils einer der Haltearme (23) bei sich in der ersten Stellung befindlichem Kanülenträger (6) hinein ragt.

6. Sicherheitsnadelanordnung (1) nach einem der vorhergehenden Ansprüche, **dadurch gekennzeichnet, dass** sich das proximale Ende (8) des Kanülenträgers (6) in Axialrichtung in den Halteansatz (14) hinein erstreckt und im Halteansatz (14) auf der dem Basiskörper (2) zugewendeten Seite über eine Teillänge zwischen dem Kanülenträger (6) und dem Halteansatz (14) eine insbesondere rohrförmig ausgebildete Aufnahmeöffnung (18) für das Stellelement (15) angeordnet ist.

7. Sicherheitsnadelanordnung (1) nach einem der vorhergehenden Ansprüche, **dadurch gekennzeichnet, dass** der Kanülenträger (6) in seiner zweiten relativen Stellung bezüglich des Basiskörpers (2) mittels einer im Bereich des proximalen Endes (4) des Basiskörpers (2) angeordneten Arretiervorrichtung in Axialrichtung relativ bezüglich des Basiskörpers (2) arretiert an diesem gehalten ist.

8. Sicherheitsnadelanordnung (1) nach einem der vorhergehenden Ansprüche, **dadurch gekennzeichnet, dass** am Basiskörper (2) von diesem in Radialrichtung abstehende Flügel (28) angeordnet sind.

9. Sicherheitsnadelanordnung (1) nach Anspruch 8, **dadurch gekennzeichnet, dass** die Flügel (28) jeweils in gleicher Richtung vom Basiskörper (2) vorragen als die diametral zueinander an den Haltearmen (23) angeordneten Vorsprünge (22).

10. Sicherheitsnadelanordnung (1) nach einem der vorhergehenden Ansprüche, **dadurch gekennzeichnet, dass** an einer Oberfläche des Kanülenträgers (6) zumindest ein als Vorsprung oder Ansatz ausgebildetes Bremselement (31) angeordnet oder ausgebildet ist.

11. Sicherheitsnadelanordnung (1) nach Anspruch 10, **dadurch gekennzeichnet, dass** mehrere Bremselemente (31) vorgesehen sind und die über den Kanülenträger (6) vorragenden Bremselemente (31) mit der im Basiskörper (2) angeordneten Durchgangsöffnung (5) zur Reduzierung der Verstellgeschwindigkeit zwischen dem nahezu bis vollständig ortsfesten Basiskörper (2) und dem dazu mittels des Stellelements (15) von der ersten Stellung in die zweite Stellung verlagerbaren Kanülenträger (6) zusammenwirken.

12. Sicherheitsnadelanordnung (1) nach Anspruch 10 oder 11, **dadurch gekennzeichnet, dass** die Bremselemente (31) zumindest im Bereich des proximalen Endes (8) des Kanülenträgers (6) angeordnet oder ausgebildet sind.

13. Sicherheitsnadelanordnung (1) nach einem der Ansprüche 10 bis 12, **dadurch gekennzeichnet, dass** die Bremselemente (31) über die gesamte axiale Baulänge des Kanülenträgers (6) vorgesehen sind.

## Claims

1. Safety needle arrangement (1) comprising a main part (2) having a distal end (3) and a proximal end (4), an orifice (5) extending therethrough between the distal end (3) of the main part (2) and the proximal end (4) of the main part (2), a cannula support (6) having a distal end (7) and a proximal end (8), a flow orifice (9) extending between the distal end (7) of the cannula support (6) and the proximal end (8) of the cannula support (6), and the cannula support (6) is accommodated in the orifice (5) of the main part (2) so as to be displaceable in the axial direction, a cannula (10) having a distal end (11) and a proximal end (12), a flow passage (13) extending inside the cannula (10) between the distal end (11) of the cannula (10) and the proximal end (12) of the cannula (10), the proximal end (12) of the cannula (10) being retained on the distal end (7) of the cannula support (6), an adjusting element (15) which is operatively arranged between the main part (2) and the cannula support (6), and the adjusting element (15) automatically moves the cannula support (6) together with the cannula (10) retained thereon from a first position, in which first position the cannula (10) projects beyond the distal end (3) of the main part (2), into a second position, in which second position at least the distal end (11) of the cannula (10) is covered by the main part (2), a locking device (19) having multiple first and second locking elements (20, 21), which locking device (19) locks the first relative position of the cannula support (6) together with the cannula (10) relative to the main part (2) when the first and second locking elements (20, 21) are in a mutually engaging position, and the first locking elements (20) are disposed in the region of the proximal end (4) of the main part (2) and the second locking elements (21) are disposed in the region of the proximal end (8) of the cannula support (6), **characterized in that** the first locking elements (20) are formed by projections (22) disposed diametrically opposite one another, which projections (22) are respectively disposed on a retaining arm (23) that is displaceable in the radial direction, and the second locking elements (21) are formed by recesses (24) disposed diametrically opposite one another.

2. Safety needle arrangement (1) according to claim 1, **characterized in that** the retaining arms (23) are respectively connected to the main part (2) in a flexible and resilient arrangement on linking sections (26) at the end remote from the projections (22) disposed thereon.

3. Safety needle arrangement (1) according to claim 1 or 2, **characterized in that** the projections (22) respectively project out from the retaining arms (23) in the radial direction towards the side remote from the orifice (5) disposed in the main part (2), and the retaining arms (23), in terms of their axial extension between their linking sections (26) and projections (22), are disposed respectively spaced apart from the main part (2).

4. Safety needle arrangement (1) according to one of the preceding claims, **characterized in that** a holder piece (14) is provided or disposed on the cannula support (6) in the region of its proximal end (8), in which holder piece (14) the recesses (24) constituting the second locking elements (21) are disposed.

5. Safety needle arrangement (1) according to claim 4, **characterized in that** the holder piece (14) has diametrically opposite insertion slots (25) in the region of its distal end, into which insertion slots (25) one of the retaining arms (23) respectively extends when the cannula support (6) is disposed in the first position.

6. Safety needle arrangement (1) according to one of the preceding claims, **characterized in that** the proximal end (8) of the cannula support (6) extends in the axial direction into the holder piece (14), and a housing orifice (18) for the adjusting element (15), which is tubular in particular, is disposed in the holder piece (14) extending across a partial length between the cannula support (6) and the holder piece (14) on the side facing the main part (2).

7. Safety needle arrangement (1) according to one of the preceding claims, **characterized in that** the cannula support (6) is locked and retained on the main part (2) by means of a locking device disposed in the region of the proximal end (4) of the main part (2) in its second position relative to the main part (2).

8. Safety needle arrangement (1) according to one of the preceding claims, **characterized in that** wings (28) are disposed on the main part (2) extending out in the radial direction therefrom.

9. Safety needle arrangement (1) according to claim 8, **characterized in that** the wings (28) respectively extend out from the main part (2) in the same direction as the projections (22) disposed diametrically opposite one another on the retaining arms (23).

10. Safety needle arrangement (1) according to one of the preceding claims, **characterized in that** at least one brake element (31) in the form of a projection or shoulder is disposed or arranged on a surface of the cannula support (6).

11. Safety needle arrangement (1) according to claim 10, **characterized in that** several brake elements (31) are provided and the brake elements (31) projecting out from the cannula support (6) co-operate with the orifice (5) disposed in the main part (2) in order to reduce the speed of movement between the virtually to completely stationary main part (2) and the cannula support (6) displaceable relative thereto by means of the adjusting element (15) from the first position into the second position.

12. Safety needle arrangement (1) according to claim 10 or 11, **characterized in that** the brake elements (31) are disposed or arranged at least in the region of the proximal end (8) of the cannula support (6).

13. Safety needle arrangement (1) according to one of claims 10 to 12, **characterized in that** the brake elements (31) are provided across the entire axial length of the cannula support (6).

## Revendications

1. Dispositif à aiguille de sécurité (1) comprenant un corps de base (2) avec une extrémité distale (3) et une extrémité proximale (4), moyennant quoi, entre l'extrémité distale (3) du corps de base (2) et l'extrémité proximale (4) du corps de base (2), dans celle-ci, s'étend une ouverture de passage (5), un support de canule (6) avec une extrémité distale (7) et une extrémité proximale (8), moyennant quoi, entre l'extrémité distale (7) du support de canule (6) et l'extrémité proximale (8) du support de canule (6), s'étend une ouverture d'écoulement (9) et le support de canule (6) est logé dans l'ouverture de passage (5) du corps de base (2) de manière mobile dans la direction axiale, une canule (10) avec une extrémité distale (11) et une extrémité proximale (12), moyennant quoi, entre l'extrémité distale (11) de la canule (10) et l'extrémité proximale (12) de la canule (10), à l'intérieur de la canule (10), s'étend un canal d'écoulement (13) et l'extrémité proximale (12) de la canule (10) est maintenue contre l'extrémité distale (7) du support de canule (6), un élément de réglage (15), qui est disposé de manière opérationnelle entre le corps de base (2) et le support de canule (6) et l'élément de réglage (15) déplace automatiquement le support de canule (6), conjointement avec la canule (10) maintenue contre lui, d'une première position, dans laquelle la canule (10) dépasse de l'extrémité distale (3) du corps de base (2), vers une deuxième position, dans laquelle au moins l'extrémité distale (11) de la canule (10) est recouverte par le corps de base (2), un dispositif de verrouillage (19) avec plusieurs premiers et deuxièmes éléments de verrouillage (20, 21), ce dispositif de verrouillage (19) fixant, dans une position d'emboîtement mutuel des premiers et deuxièmes éléments de verrouillage (20, 21), la première position relative du support de canule (6) conjointement avec la canule (10) par rapport au corps de base (2), et les premiers éléments de verrouillage (20) étant disposé au niveau de l'extrémité proximale (4) du corps de base (2) et les deuxièmes éléments de verrouillage (21) étant disposés au niveau de l'extrémité proximale (8) du support de canule (6), **caractérisé en ce que** les premiers éléments de verrouillage (20) sont constitués de saillies (22) disposées de manière diamétralement opposée entre elles, ces saillies (22) étant disposées chacune sur un bras de maintien (23) mobile dans la direction radiale et les deuxième éléments de verrouillage (21) sont constitués d'évidements (24) disposés de manière diamétralement opposée entre eux.

2. Dispositif à aiguille de sécurité (1) selon la revendication 1, **caractérisé en ce que** les bras de maintien (23) sont reliés de manière élastique et articulée chacun sur le côté opposé aux saillies (22) disposées dessus, au niveau de portions d'articulation (26), avec le corps de base (2).

3. Dispositif à aiguille de sécurité (1) selon la revendication 1 ou 2, **caractérisé en ce que** les saillies (22) dépassent chacune, sur le côté opposé à l'ouverture de passage (5) disposé dans le corps de base (2), des bras de maintien (23) dans la direction radiale et les bras de maintien (23) sont disposés, dans leur extension axiale, entre leurs portions d'articulation (26) et les saillies (22), à une certaine distance du corps de base (2).

4. Dispositif à aiguille de sécurité (1) selon l'une des revendications précédentes, **caractérisé en ce que**, sur le support de canule (6), au niveau de son extrémité proximale (8), est disposé ou réalisé un embout de maintien (14), les évidements (24) constituant les deuxièmes éléments de verrouillage (21) étant disposés dans cet embout de maintien (14).

5. Dispositif à aiguille de sécurité (1) selon la revendication 4, **caractérisé en ce que** l'embout de maintien (14) comprend, au niveau de son extrémité distale, des fentes d'insertion (25) disposées de manière opposée diamétralement, un des bras de maintien (23) dépassant dans ces fentes d'insertion (25) lorsque le support de canule (6) se trouve dans la première position.

6. Dispositif à aiguille de sécurité (1) selon l'une des revendications précédentes, **caractérisé en ce que** l'extrémité proximale (8) du support de canule (6) s'étend à l'intérieur de l'embout de maintien (14) dans la direction axiale et, dans l'embout de maintien (14), sur le côté orienté vers le corps de base (2), sur une longueur partielle entre le support de canule (6) et l'embout de maintien (14) est disposée une ouverture de logement (18), plus particulièrement de forme tubulaire, pour l'élément de réglage (15).

7. Dispositif à aiguille de sécurité (1) selon l'une des revendications précédentes, **caractérisé en ce que** le support de canule (6) est maintenu de manière bloquée dans sa deuxième position relative par rapport au corps de base (2) au moyen d'un dispositif de blocage disposé au niveau de l'extrémité proximale (4) du corps de base (2), dans la direction axiale par rapport au corps de base (2) sur celui-ci.

8. Dispositif à aiguille de sécurité (1) selon l'une des revendications précédentes, **caractérisé en ce que**, sur le corps de base (2), sont disposées des ailettes (28) qui partent de celui-ci dans la direction radiale.

9. Dispositif à aiguille de sécurité (1) selon la revendication 8, **caractérisé en ce que** les ailettes (28) dépassent chacune du corps de base (2) dans la même direction que les saillies (22) disposées de manière diamétralement opposée sur les bras de maintien (23).

10. Dispositif à aiguille de sécurité (1) selon l'une des revendications précédentes, **caractérisé en ce que**, sur une surface du support de canule (6), est disposé ou réalisé au moins un élément de freinage (31) conçu comme une saillie ou en embout.

11. Dispositif à aiguille de sécurité (1) selon la revendication 10, **caractérisé en ce que** plusieurs éléments de freinage (31) sont prévus et les éléments de freinage (31) dépassent du support de canule (6) interagissent avec l'ouverture de passage (5) disposée dans le corps de base (2) afin de réduire la vitesse de déplacement entre le corps de base (2), presque à entièrement stationnaire, et le support de canule (6) mobile, au moyen de l'élément de réglage (15), de la première position vers la deuxième position.

12. Dispositif à aiguille de sécurité (1) selon la revendication 10 ou 11, **caractérisé en ce que** les éléments de freinage (31) sont disposés ou réalisés au moins au niveau de l'extrémité proximale (8) du support de canule (6).

13. Dispositif à aiguille de sécurité (1) selon l'une des revendications 10 à 12, **caractérisé en ce que** les éléments de freinage (31) sont prévus sur toute la longueur axiale du support de canule (6).
